# EUROPEAN PATENT APPLICATION

(11) **EP 4 640 219 A1**
(43) Date of publication of application: **29.10.2025**
(21) Application number: 23907200.2
(22) Date of filing: 22.12.2023
(51) Int. Cl.: A61K 31/4418, A61K 9/16, A61K 9/20, A61K 47/10, A61K 47/12, A61K 47/14, A61K 47/20, A61K 47/26, A61K 47/34, A61P 25/00, A61P 25/14, A61P 25/16, A61P 25/18, A61P 25/28, A61P 43/00

(54) **CAPSULE OR GRANULES CONTAINING PDE4 INHIBITOR**

(30) Priority: 23.12.2022 JP 2022207385
(71) Applicant: Shionogi & Co., Ltd., Osaka-shi, Osaka 541-0045 (JP)
(72) Inventor: MIZUTANI, Naoya, Osaka-shi, Osaka 541-0045 (JP); YOKOYAMA, Reiji, Osaka-shi, Osaka 541-0045 (JP); TANAKA, Takahiro, Osaka-shi, Osaka 541-0045 (JP); MIYOSHI, Daichi, Osaka-shi, Osaka 541-0045 (JP)
(74) Representative: Vossius & Partner Patentanwälte Rechtsanwälte mbB
(86) International application number: PCT/JP2023/046217
(87) International publication number: WO 2024/135840

(57) **Abstract**

A capsule or granule formulation comprising: a compound represented by Formula (I): 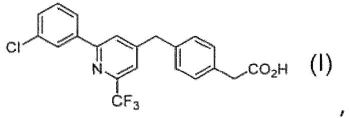 a pharmaceutically acceptable salt thereof, or a solvate thereof is provided.

When the capsule or granule formulation comprises a surfactant, the capsule or granule formulation comprising the compound represented by Formula (I), having excellent dissolution properties is provided.

## Description

### Technical Field

The present invention relates to a capsule or granule formulation comprising: a compound represented by Formula (I): 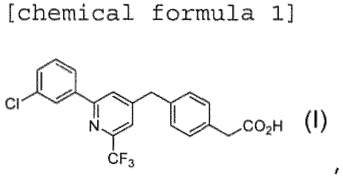 a pharmaceutically acceptable salt thereof, or a solvate thereof (hereinafter referred to as a compound represented by Formula (I) or the like); and a surfactant. More particularly, the present invention relates to a capsule or granule formulation comprising the surfactant as a lubricant.

### Background Art

PATENT DOCUMENT 1 indicates that compound represented by Formula (I) and the like exhibit the inhibitory activity of PDE4 (phosphodiesterase 4) and are useful for the treatment of certain diseases and disorders of the nervous system. Central nervous system diseases for which PDE4 inhibition is useful include cortical dementia, including Alzheimer's disease, dementia with AIDS (HIV dementia), and mild cognitive impairment (MCI). Neurodegenerative diseases for which PDE4 inhibition is useful include, in the case of hypoxia, hypoglycemia, epilepsy, and central nervous system (CNS) trauma (such as spinal cord and head injuries), hyperbaric oxygen attack and toxicity, dementia, e.g., presenile dementia, and HIV-associated neurodegenerative disorders (HAND), cachexia, Sydenham chorea, Huntington's disease, Parkinson's disease, Amyotrophic Lateral Sclerosis (ALS), Korsakoff syndrome, and neurological denaturation or neuronecrosis in disorders, such as dysfunction associated with cerebrovascular disorders.

PATENT DOCUMENT 2 describes crystals of compound represented by Formula (I) or the like.

However, none of the pieces of patent document discloses a formulation of the present invention.

### Citation List

### Patent Document

PATENT DOCUMENT 1: WO 2014/066659
PATENT DOCUMENT 2: WO 2020/214874

### Summary of Invention

### Technical Problem

It is an object of the present invention to provide a capsule or granule formulation comprising a compound represented by Formula (I) or the like.

### Solution to Problem

The present inventors have found that, in production of the capsule or granule formulation comprising the compound represented by Formula (I) or the like, the capsule or granule formulation having excellent dissolution properties can be produced by using a surfactant, and have completed the capsule or granule formulation comprising the compound represented by Formula (I) and the like and a surfactant, of the present invention.

The present inventors have found a capsule or granule formulation comprising the compound represented by Formula (I) or the like shown below.
(1) A capsule or granule formulation comprising a compound represented by Formula (I): 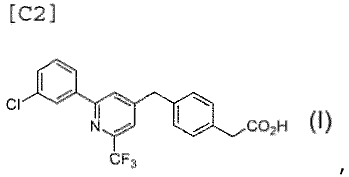 a pharmaceutically acceptable salt thereof, or a solvate thereof, and a surfactant.
(2) The capsule or granule formulation according to the item (1) above, wherein the surfactant is included as a lubricant.
(3) The capsule or granule formulation according to the item (1) or (2) above, wherein the surfactant is at least one selected from the group consisting of sucrose fatty acid ester, sodium lauryl sulfate, polysorbate, macrogol, glycerol monostearate, glycerin fatty acid ester, polyoxyl stearate, cetanol, and polyoxyethylene polyoxypropylene glycol.
(4) The capsule or granule formulation according to the item (3) above, wherein the surfactant is a sucrose fatty acid ester.
(5) The capsule or granule formulation according to any one of the items (1) to (4) above, wherein the capsule or granule does not comprise magnesium stearate, talc, calcium stearate, stearic acid, or carnauba wax.
(6) The capsule according to any one of the items (1) to (5) above, wherein the capsule is a capsule of No. 3 or smaller.
(7) The capsule according to any one of the items (1) to (6) above, wherein the compound represented by Formula (I), a pharmaceutically acceptable salt thereof, or a solvate thereof is included in 5 mg, 10 mg, 15 mg, or 25 mg.
(8) The capsule or granule formulation according to any one of the items (1) to (7) above, further comprising a filler (preferably crystalline cellulose).
(9) The capsule or granule formulation according to any one of the items (1) to (7) above, further comprising a binder (preferably, povidone).
(10) The capsule or granule formulation according to any one of the items (1) to (7) above, further comprising a disintegrant (preferably croscarmellose sodium).
(11) The capsule or granule formulation according to any one of the items (1) to (7) above, further comprising an filler and a binder (preferably crystalline cellulose and povidone).
(12) The capsule or granule formulation according to any one of the items (1) to (7) above, further comprising an filler and a disintegrant (preferably crystalline cellulose and croscarmellose sodium).
(13) The capsule or granule formulation according to any one of the items (1) to (7) above, further comprising a binder and a disintegrant (preferably povidone and croscarmellose sodium) .
(14) The capsule or granule formulation according to any one of the items (1) to (7) above, further comprising an filler, a binder, and a disintegrant (preferably crystalline cellulose, povidone, and croscarmellose sodium).
(15) The capsule or granule formulation according to any one of the items (1) to (14) above, further comprising a fluidizer (preferably light anhydrous silicic acid).
(16) The capsule or granule formulation according to any one of the above items (1) to (15), further comprising D-mannitol. Advantageous Effects of Invention

The present invention provides a capsule or granule formulation comprising a compound represented by Formula (I) and the like, which are excellent in dissolution properties.

### Brief Description of the Drawings

[Fig. 1]

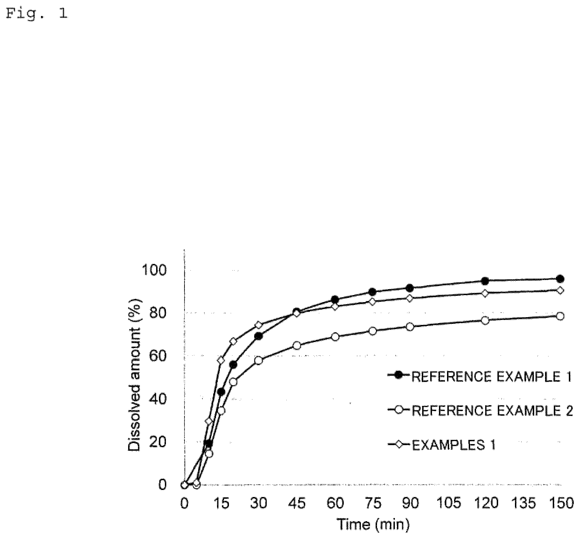

Fig. 1 shows the results of the dissolution test of the capsules in Reference Examples 1 and 2 and Example 1. The vertical axis indicates the dissolution rate (unit: %), and the horizontal axis indicates time (unit: min).

### Description of Embodiments

The present invention will be described in detail below.

The present invention relates to a capsule or granule formulation comprising a compound represented by Formula (I) or the like.

One aspect of the present invention is directed to a capsule or granule formulation comprising a compound represented by Formula (I) or the like and a surfactant.

Also, one aspect of the present invention is directed to the capsule or granule formulation comprising the surfactant as a lubricant.

The surfactant is at least one selected from the group consisting of sucrose fatty acid ester, sodium lauryl sulfate, polysorbate, macrogol, glycerol monostearate, glycerin fatty acid ester, polyoxyl stearate, cetanol, and polyoxyethylene polyoxypropylene glycol. The surfactant is particularly preferably sucrose fatty acid ester.

The surfactant can be used in an amount of 1 to 10 wt%, relative to the total weight of the composition with which the capsule is filled. The surfactant is used in an amount of preferably 2 to 6 wt%. The surfactant is used in an amount of particularly preferably 4.4 to 4.8 wt%. The surfactant is used in an amount of even more preferably 4.7 wt%.

In the granule of the present invention, the surfactant can be used in an amount of 1 to 10 wt%, relative to the total weight of the granule. The surfactant is used in an amount of preferably 2 to 6 wt%. The surfactant is used in an amount of particularly preferably 4.4 to 4.8 wt%. The surfactant is used in an amount of even more preferably 4.7 wt%.

As the size of the capsule to be used for the capsule formulation, capsules of No. 2 to 5 specified by the capsule size standard of Japanese Pharmacopoeia 18th Edition are preferable. The capsule is preferably a capsule of No. 3 or smaller. The capsule is particularly preferably a capsule of No. 3 or No. 4. The capsule is further preferably a capsule of No. 3.

In capsule size standard of the Japanese Pharmacopoeia 18th Edition, the capsule fill weight (g) of the capsule of No. 2 is 0.2, the capsule fill weight (g) of the capsule of No. 3 is 0.12, the capsule fill weight (g) of the capsule of No. 4 is 0.06, and the capsule fill weight (g) of the capsule of No. 5 is 0.03.

For example, each capsule may be the following. The capsule of No. 2 used can be a capsule having a body volume (ml) of about 0.37, an overall length when assembled (mm) of about 18.0, and a body outer diameter (mm) of about 6.08. The capsule of No. 3 used can be a capsule having a body volume (ml) of about 0.27, an overall length when assembled (mm) of about 15.8, and a body outer diameter (mm) of about 5.56. The capsule of No. 4 used can be a capsule having a body volume (ml) of about 0.21, an overall length when assembled (mm) of about 14.5, and a body outer diameter (mm) of about 5.06. The capsule of No. 5 used can be a capsule having a body volume (ml) of about 0.13, an overall length when assembled (mm) of about 11.4, and a body outer diameter (mm) of about 4.66. The above is illustrative and not limiting each capsule.

Examples of the capsule material used for the capsule include hypromellose, gelatin, and pullulan. The capsule material is preferably hypromellose and gelatin. The capsule material is particularly preferably hypromellose.

One aspect of the present invention is directed to the capsule or granule formulation, wherein the capsule or granule formulation does not comprise magnesium stearate, talc, calcium stearate, stearic acid, or carnauba wax. In the present invention, since the surfactant functions as a lubricant, it is not necessary to use the above-described general-purpose lubricant.

In the capsule or granule formulation of the present invention, an additive used in the pharmaceutical field can be used in addition to the surfactant. For example, the capsule or granule formulation may further comprise a filler, a binder, a disintegrant, a fluidizer, and the like. Examples of the filler used include mannitol (e.g., D-mannitol), microcrystalline cellulose, crystalline cellulose, lactose, corn starch, potato starch, and the like. . The filler is particularly preferably mannitol (e.g., D-mannitol), crystalline cellulose, or microcrystalline cellulose.

Examples of the binder used include povidone, crystalline cellulose, hydroxypropyl cellulose, hypromellose, low substituted hydroxypropylcellulose, and the like. The binder is particularly preferably povidone or crystalline cellulose.

Examples of the disintegrant used include croscarmellose sodium, low substituted hydroxypropylcellulose, carmellose, carmellose calcium, partially pregelatinized starch, sodium carboxymethylstarch, crospovidone, polyvinyl polypyrrolidone, and the like. The disintegrant is preferably croscarmellose sodium.

Examples of the fluidizer used include light anhydrous silicic acid, hydrated silicon dioxide, magnesium aluminometasilicate, talc, and the like. The fluidizer is particularly preferably light anhydrous silicic acid.

The pharmaceutically acceptable salt of the compound represented by Formula (I) is not particularly limited, and examples thereof include sodium salts, potassium salts, and calcium salts.

Examples of the solvate of the compound represented by Formula (I) or a pharmaceutically acceptable salt thereof include hydrates, ethanol solvate, dioxane solvate, and dichloromethane solvate. The solvate is preferably hydrate. The method for producing dioxane solvate and dichloromethane solvate is described in PATENT DOCUMENT 2.

The compound represented by Formula (I) or a salt thereof may include adhesive water. In this case, 0.5 to 1.5 wt% of water may adhere to the compound represented by Formula (I) or a salt thereof. For example, 1.1 wt% of water may adhere.

In the capsule of the present invention, the compound represented by Formula (I) and the like can be used in an amount of 1 to 40 wt%, relative to the total weight of the composition with which the capsule is filled. The amount is preferably 4 to 30 wt%. The amount is particularly preferably 15 to 25 wt%. The amount is even preferably 20.8 wt%.

In the granule of the present invention, the compound represented by Formula (I) and the like can be used in an amount of 1 to 40 wt%, relative to the total weight of the granule. The amount is preferably 4 to 30 wt%. The amount is particularly preferably 15 to 25 wt%. The amount is even preferably 20.8 wt%.

The granule formulation of the present invention and the granule in the capsule of the present invention are prepared by mixing and granulating the compound represented by Formula (I) and the like, a filler, and a binder to produce granules, and then mixing the granules with granules produced by mixing and granulating a disintegrant, a fluidizer, and a surfactant. As a machine for the mixing, a V-type mixer, a container blender, or a stirring granulator can be used. As a machine for the granulating, a fluidized bed granulating machine or a stirring granulating machine can be used.

Capsules are filled with the granules prepared above to produce capsules of the present invention.

The capsule or granule formulation of the present invention may contain, but are not particularly limited to, 5 to 30 mg of the compound represented by Formula (I) and the like. The amount is preferably 5 mg, 10 mg, 15 mg, or 25 mg, even more preferably 25 mg.

The capsule or granule formulation of the present invention can be administered orally. Without particular limitation, 5 to 100 mg/day of the compound represented by Formula (I) can be administered. The administration amount is preferably 10 to 80 mg/day. The administration amount is particularly preferably 25 to 50 mg/day.

The total weight of the capsule formulation of the present invention is not particularly limited, and is 50 to 250 mg, more preferably 160 to 170 mg, particularly preferably 166 mg. The capsule or granule formulation of the present invention can be used for applications described in PATENT DOCUMENT 1. For example, it can be used for the prevention and/or treatment of certain diseases and disorders of the nervous system. Specifically, the present invention can be used for, in the case of cortical dementia, including Alzheimer's disease, dementia with AIDS (HIV dementia), mild cognitive impairment (MCI), hypoxia, hypoglycemia, epilepsy, and central nervous system (CNS) trauma (such as spinal cord and head injuries), the prevention and/or treatment of hyperbaric oxygen attack and toxicity, dementia, e.g., presenile dementia, and HIV-associated neurodegenerative disorders (HAND), cachexia, Sydenham chorea, Huntington's disease, Parkinson's disease, Amyotrophic Lateral Sclerosis (ALS), Korsakoff syndrome, and neurological denaturation or neuronecrosis in disorders, such as dysfunction associated with cerebrovascular disorders. The capsule or granule formulation of the present invention can improve cognitive and memory functions in central nervous system (CNS) disorders that are difficult to treat, such as fragile X syndrome (FXS), Alzheimer's-type dementia and other dementias, learning/developmental disorders, and schizophrenia.

### Examples

The present invention will be specifically described below with reference to the examples, but the scope of the present invention is not limited to these examples.

### (Reference Example 1)

Magnesium stearate was used to produce granules containing the compound represented by Formula (I) and produce No. 1 capsules filled with the granules. The method for producing is described below.

The compound represented by Formula (I), crystalline cellulose, mannitol, and povidone were sieved, then mixed, and granulated with water. Granules granulated and sized were mixed with crystalline cellulose, croscarmellose sodium, light anhydrous silicic acid, and magnesium stearate, thereby obtaining granules. No. 1 capsule shells were filled with the granules obtained to produce capsules. As No. 1 capsule shells, hypromellose capsules (V-Caps^{(R)} Plus, manufactured by Ronza) were used.

The proportion of the composition with which the capsules are filled relative to the total weight of the granules with which the capsules are filled are shown below.

**[Table 1]**

| Components | Amount |
|---|---|
| Compound represented by Formula (I) | 12.6% |
| Mannitol | 37.5% |
| Crystalline cellulose | 38.4% |
| Povidone | 5.0% |
| Croscarmellose sodium | 5.0% |
| Light anhydrous silicic acid | 1.0% |
| Magnesium stearate | 0.5% |

### (Reference Example 2)

Magnesium stearate was used to produce granules containing the same amount of the compound represented by Formula (I) as in Reference Example 1, and No. 3 capsules filled with the granules were produced. The method for producing is described below.

The compound represented by Formula (I), crystalline cellulose, D-mannitol, and povidone were sieved, then mixed, and granulated with water. Granules granulated and sized were mixed with croscarmellose sodium, light anhydrous silicic acid, and magnesium stearate, thereby obtaining granules for filling. No. 3 capsule shells were filled with the granules obtained to produce capsules. As No. 3 capsule shells, hypromellose capsules (V-Caps^{(R)} Plus, manufactured by Ronza) were used.

The proportion of the composition with which the capsules are filled relative to the total weight of the granules with which the capsules are filled are shown below.

**[Table 2]**

| Components | Amount |
|---|---|
| Compound represented by Formula (I) | 21.7% |
| D-Mannitol | 41.0% |
| Crystalline cellulose | 24 . 7% |
| Povidone | 6.0% |
| Croscarmellose sodium | 5.0% |
| Light anhydrous silicic acid | 1.0% |
| Magnesium stearate | 0.5% |

### (Example 1)

Sucrose fatty acid ester was used to produce granules containing the same amount of the compound represented by Formula (I) as in Reference Example 2, and No. 3 capsules filled with the granules were produced. The method for producing is described below.

The compound represented by Formula (I), crystalline cellulose, D-mannitol, and povidone were sieved, then mixed, and granulated with water. Granules granulated and sized were mixed with croscarmellose sodium, light anhydrous silicic acid, and sucrose fatty acid ester, thereby obtaining granules for filling. No. 3 capsule shells were filled with the granules obtained to produce capsules. As No. 3 capsule shells, hypromellose capsules (V-Caps^{(R)} Plus, manufactured by Ronza) were used.

The proportion of the composition with which the capsules are filled relative to the total weight of the granules with which the capsules are filled are shown below.

**[Table 3]**

| Components | Amount |
|---|---|
| Compound represented by Formula (I) | 20.8% |
| D-Mannitol | 39.3% |
| Crystalline cellulose | 23.7% |
| Povidone | 5.8% |
| Croscarmellose sodium | 4.8% |
| Light anhydrous silicic acid | 0.9% |
| Sucrose fatty acid ester | 4.7% |

### (Test Example 1) Dissolution Test

A dissolution test was performed on the capsules of Reference Examples 1 and 2 and Example 1. The test was performed using 900 mL of pH 6.8 phosphate buffer as a test solution and using the paddle method at 75 revolutions per minute. For each one of the capsules of Reference Examples 1 and 2 and Example 1, the test was started, and 10 mL of the dissolved solution was accurately taken after 5, 10, 15, 20, 30, 45, 60, 75, 90, 120 and 150 minutes and filtered through a membrane filter with a pore size of 0.45 µm. The obtained solutions except for first 8 mL of the filtrate was used as test solutions. About 28 mg of the standard of the compound represented by Formula (I) was precisely weighed, dissolved in acetonitrile, and made up to precisely 50 mL. 5 mL of this liquid was accurately weighed and water/acetonitrile (1:1) was added to 50 mL as a standard solution. Each 4 µL of the test solutions and standard solution was taken accurately and tested by liquid chromatography under the following conditions, and the peak area of each compound represented by Formula (I) was measured.

### Test conditions

Detector: Ultraviolet absorptiometer (measurement wavelength: 248 mm)
Column: A stainless steel tube with an inner diameter of 2.1 mm and a length of 5 cm was charged with 1.7 µm octadecylsilylated silica gel for liquid chromatography (Acquity UPLC BEH C18 (Waters) or equivalent)
Column temperature: Constant temperature around 40°C
Mobile phase A: Water containing 0.1 vol% formic acid/acetonitrile mixture (9:1)
Mobile phase B: An acetonitrile solution for liquid chromatography containing 0.1 vol% formic acid Gradient program

**[Table 4]**

| Time (min) | Flow rate (mL/min) | Mobile phase A (%) | Mobile phase B (%) |
|---|---|---|---|
| 0.00 | 0.5 | 100 | 0 |
| 1.00 | 0.5 | 100 | 0 |
| 6.00 | 0.5 | 0 | 100 |
| 6.50 | 0.5 | 0 | 100 |
| 6.51 | 0.5 | 100 | 0 |
| 7.50 | 0.5 | 100 | 0 |

Liquid volume: Adjusted so that the retention time of the compound represented by Formula (I) is about 5 minutes.

As a result of the above test, the capsules of Reference example 2 showed poorer dissolution and lower dissolution rate than the capsules of Reference example 1. The capsule of Example 1 showed similar rapid dissolution behavior as the capsule of Reference example 1. The results are shown in Fig. 1.

### (Example 2)

Capsules containing 2.9% sucrose fatty acid ester were prepared in the same manner as in Example 1. The weight of the compound represented by Formula (I) is the same as in Example 1.

The proportion of the composition with which the capsules are filled relative to the total weight of the granules with which the capsules are filled are shown below.

**[Table 5]**

| Components | Amount |
|---|---|
| Compound represented by Formula (I) | 21.2% |
| D-Mannitol | 40.1% |
| Crystalline cellulose | 24.1% |
| Povidone | 5.9% |
| Croscarmellose sodium | 4.9% |
| Light anhydrous silicic acid | 0.9% |
| Sucrose fatty acid ester | 2.9% |

### (Example 3)

Capsules containing 1% sucrose fatty acid ester were prepared in the same manner as in Example 1. The weight of the compound represented by Formula (I) is the same as in Example 1.

The proportion of the composition with which the capsules are filled relative to the total weight of the granules with which the capsules are filled are shown below.

**[Table 6]**

| Components | Amount |
|---|---|
| Compound represented by Formula (I) | 21.7% |
| D-Mannitol | 40.9% |
| Crystalline cellulose | 24.6% |
| Povidone | 6.0% |
| Croscarmellose sodium | 5.0% |
| Light anhydrous silicic acid | 1.0% |
| Sucrose fatty acid ester | 1.0% |

A dissolution test was performed on the capsules of Examples 2 and 3 in the same manner as in Test Example 1. As a result, a good dissolution behavior was shown similar to the capsule of Example 1.

### (Example 4)

In the same manner as in Example 1, a capsule containing 12.5% compound represented by Formula (I) was prepared. The weight of the composition with which the capsules are filled in the total weight of the granules with which the capsules are filled is the same as in Example 1.

The proportion of the composition with which the capsules are filled relative to the total weight of the granules with which the capsules are filled are shown below.

**[Table 7]**

| Components | Amount |
|---|---|
| Compound represented by Formula (I) | 12.5% |
| D-Mannitol | 47.7% |
| Crystalline cellulose | 23.7% |
| Povidone | 5.8% |
| Croscarmellose sodium | 4.8% |
| Light anhydrous silicic acid | 0.9% |
| Sucrose fatty acid ester | 4.7% |

### (Example 5)

In the same manner as in Example 1, a capsule containing 8.3% compound represented by Formula (I) was prepared. The weight of the composition with which the capsules are filled in the total weight of the granules with which the capsules are filled is the same as in Example 1.

The proportion of the composition with which the capsules are filled relative to the total weight of the granules with which the capsules are filled are shown below.

**[Table 8]**

| Components | Amount |
|---|---|
| Compound represented by Formula (I) | 8.3% |
| D-Mannitol | 51.8% |
| Crystalline cellulose | 23.7% |
| Povidone | 5.8% |
| Croscarmellose sodium | 4.8% |
| Light anhydrous silicic acid | 0.9% |
| Sucrose fatty acid ester | 4.7% |

### (Example 6)

In the same manner as in Example 1, a capsule containing 4.2% compound represented by Formula (I) was prepared. The weight of the composition with which the capsules are filled in the total weight of the granules with which the capsules are filled is the same as in Example 1.

The proportion of the composition with which the capsules are filled relative to the total weight of the granules with which the capsules are filled are shown below.

**[Table 9]**

| Components | Amount |
|---|---|
| Compound represented by Formula (I) | 4.2 |
| D-Mannitol | 56.0% |
| Crystalline cellulose | 23.7% |
| Povidone | 5.8% |
| Croscarmellose sodium | 4.8% |
| Light anhydrous silicic acid | 0.9% |
| Sucrose fatty acid ester | 4.7% |

A dissolution test was performed on the capsules of Examples 4 to 6 in the same manner as in Test Example 1. As a result, a good dissolution behavior was shown similar to the capsule of Example 1.

### Industrial Applicability

The capsule or granule formulation of the present invention exhibits excellent dissolution properties, and thus is useful in the prevention and/or treatment of diseases of certain diseases and disorders of the nervous system.

## Claims

1. A capsule or granule formulation comprising: a compound represented by Formula (I): 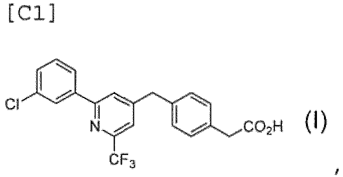
a pharmaceutically acceptable salt thereof, or a solvate thereof; and
a surfactant.

2. The capsule or granule formulation according to claim 1, wherein the surfactant is included as a lubricant.

3. The capsule or granule formulation according to claim 1 or 2, wherein the surfactant is at least one selected from the group consisting of sucrose fatty acid ester, sodium lauryl sulfate, polysorbate, macrogol, glycerol monostearate, glycerin fatty acid ester, polyoxyl stearate, cetanol, and polyoxyethylene polyoxypropylene glycol.

4. The capsule or granule formulation according to claim 3, wherein the surfactant is a sucrose fatty acid ester.

5. The capsule or granule formulation according to any one of claims 1 to 4, wherein the capsule or granule does not comprise magnesium stearate, talc, calcium stearate, stearic acid, or carnauba wax.

6. The capsule formulation according to any one of claims 1 to 5, wherein the capsule is a capsule of No. 3 or smaller specified by a capsule size standard of Japanese Pharmacopoeia 18th Edition.

7. The capsule formulation according to any one of claims 1 to 6, wherein the compound represented by Formula (I), a pharmaceutically acceptable salt thereof, or a solvate thereof is included in 5 mg, 10 mg, 15 mg, or 25 mg.
